# EUROPEAN PATENT APPLICATION

(11) **EP 4 206 309 A1**
(43) Date of publication of application: **05.07.2023**
(21) Application number: 21218378.4
(22) Date of filing: 30.12.2021
(51) Int. Cl.: C11D 3/00, C11D 3/04, C11D 3/22, C11D 3/386, C11D 7/10, C11D 7/26, C11D 17/06

(54) **PROTEIN PARTICLES WITH IMPROVED WHITENESS**

(71) Applicant: Novozymes A/S, 2880 Bagsvaerd (DK)
(72) Inventor: SIMONSEN, Ole, 2880 Bagsvaerd (DK); HELLSTRAND, Erik, 2880 Bagsvaerd (DK); MOELLER, Kristoffer Hauberg, 2880 Bagsvaerd (DK); SHANG, Lei, 2880 Bagsvaerd (DK)
(74) Representative: NZ EPO Representatives

(57) **Abstract**

The invention provides protein particles having improved whiteness after storage, and powder detergents comprising the particles.

## Description

### FIELD OF THE INVENTION

The present invention relates to protein (e.g. enzyme) particles exhibiting improved longterm brightness/whiteness (reduced browning), and powder detergents comprising such particles.

### BACKGROUND

The whiteness of many consumer powder products, such as powder detergents and certain foods, is generally associated with efficiency and/or high quality. But when the particles of such powder products comprise both protein and reducing sugars, they may develop a brown color over time, due to Maillard reactions between the reducing sugars and amino acids of the protein. This browning effect can be masked by adding whitening agents, such as titanium dioxide, which is used in huge amounts for this purpose.

However, especially titanium dioxide is suspected of having a negative environmental impact, and there is a general desire to develop whitening technologies that avoids or reduces the use of titanium dioxide.

The present invention provides a way to reduce the requirements for whitening agents in particles comprising protein and reducing sugars, used in white consumer powder products.

### SUMMARY OF THE INVENTION

The present invention provides, in a first aspect, a particle comprising
(a) a protein;
(b) a salt of sulfite, bisulfite, or metabisulfite; and
(c) a mono- or disaccharide which is a reducing sugar or can be degraded to a reducing sugar.

In a second aspect, the invention provides a particle comprising
(a) a protein;
(b) a salt of sulfite, bisulfite, or metabisulfite; and
(c) less than 0.5% w/w of titanium dioxide; preferably less than 0.1% w/w of titanium dioxide

Other aspects and embodiments of the invention are apparent from the description and examples.

Unless otherwise indicated, or if it is apparent from the context that something else is meant, all percentages are percentage by weight (% w/w).

As used herein, the term "consists essentially of" (and grammatical variants thereof), as applied to the compositions and methods of the invention, means that the compositions/methods may contain additional components so long as the additional components do not materially alter the composition/method.

As used herein, the term "essentially free of" (and grammatical variants thereof), as applied to the compositions and methods of the invention, means that the compositions/methods may contain minor amounts of the specified component so long as the amount of the component does not materially alter, or provide any material effect on, the composition/method. In an embodiment, "essentially free of" means 0% w/w.

### DETAILED DESCRIPTION

We have found that the browning associated with Maillard reactions between proteins and reducing sugars in white particles/granules can be reduced by adding a salt of sulfite, bisulfite, or metabisulfite. In this way, the amount of whitening agent - such as titanium dioxide - can be reduced or completely avoided.

When proteins are produced by fermentation, this is even more relevant, because the fermented microorganisms used to produce the proteins need sugars or sugar-precursors as a carbon source in the fermentation process. If small amounts of reducing sugars are left with the proteins after purification, this may subsequently lead to browning during storage of the protein particles.

Since the Maillard reactions occur between amino acids and reducing sugars, it is not important what kind of protein(s) the particles contain. For example, enzymes are a well-known group of proteins produced by fermentation, which are widely used as white particles in both consumer and prosumer products. Thus, the present invention is particularly useful for maintaining whiteness of enzyme particles/granules.

### Protein particles

As described above, the invention provides a particle comprising
(a) a protein;
(b) a salt of sulfite, bisulfite, or metabisulfite; and
(c) a mono- or disaccharide which is a reducing sugar or can be degraded to a reducing sugar.

Reducing sugars can react with the amino acids of the protein to produce Maillard rection products that reduce whiteness of the particle after storage. Examples of reducing sugars include glucose, fructose, lactose, and maltose. Many disaccharides, such as sucrose, (or even oligosaccharides) can be degraded by hydrolysis of the glycosidic bond to yield reducing sugars during storage, and thus effectively act as reducing sugars. For example, hydrolysis of sucrose yields glucose and fructose, which are both reducing sugars. Hydrolysis of sucrose is catalyzed by the enzyme invertase/sucrase, which is often present in fermentation products. Thus, in an embodiment, the mono- or disaccharide is selected from the group consisting of glucose, fructose, lactose, maltose, sucrose, and combinations thereof.

Preferably, the mono- or disaccharide is a reducing sugar, which may be measured as glucose equivalents using the PAHBAH assay.

In an embodiment, the particle comprises the mono- or disaccharide in an amount of more than 0.01% w/w; preferably more than 0.05% w/w.

Since the particle of the invention exhibit less browning after storage, the particle may comprise less than 0.5% w/w of titanium dioxide, preferably less than 0.1% w/w, more preferably less than 0.05% w/w, or is essentially free of titanium dioxide. Thus, the invention also provides another particle comprising
(a) a protein;
(b) a salt of sulfite, bisulfite, or metabisulfite; and
(c) less than 0.5% w/w of titanium dioxide; preferably less than 0.1% w/w of titanium dioxide.

Both particles (which are hereafter referred to as "the particles") may comprise the salt of sulfite, bisulfite, or metabisulfite in an amount of more than 0.01% w/w; preferably more than 0.05% w/w, or more than 0.1% w/w. The upper limit of the salt of sulfite, bisulfite, or metabisulfite may be (less than) 10% w/w, preferably 5% w/w or 1% w/w.

The particles may comprise the protein in an amount of 0.1-25% w/w.

It is advantageous if the protein and the salt of sulfite, bisulfite, or metabisulfite are homogenously mixed in a continuous matrix. Preferably, the particles comprise a core and one or more coating(s) surrounding the core, and the core comprises, preferably consists of, the continuous matrix.

As mentioned above, proteins produced by fermented microorganisms often contain small amounts of reducing sugars from the fermentation medium, even after purification. Thus, in an embodiment, the protein is produced by fermentation.

In a particular embodiment, the protein of the particles is an enzyme, and the amount of protein (enzyme) is active enzyme protein (AEP).

The salt of sulfite, bisulfite, or metabisulfite may be a salt of metabisulfite; preferably sodium metabisulfite or potassium metabisulfite.

Since the particles of the invention, as described above, exhibit reduced browning after storage, it is not necessary to add the same amount of whitening agent (such as titanium dioxide) as is commonly used for improving the whiteness of particles. Thus, the particles may comprise less than 0.5% w/w of titanium dioxide, less than 0.1% w/w, or be essentially free of titanium dioxide.

Likewise, the particles of the invention may exhibit reduced loss of whiteness after storage; preferably having a delta Hunter whiteness of less than 5 after storage for 8 weeks at 37°C and 70% RH. Alternatively, the particles of the invention may have a Hunter whiteness, which is more than 5 units higher after storage, as compared to the same particles comprising no salt of sulfite, bisulfite, or metabisulfite.

The particles, as described above, may be produced in a process comprising the steps of
(a) preparing a homogenous mixture of a protein produced by fermentation, and a salt of sulfite, bisulfite, or metabisulfite; and
(b) preparing a particle from the homogenous mixture.

The invention also provides a method for improving the whiteness after storage of a protein particle, comprising
(a) preparing the particle, as described above; and
(b) storing the particle for more than 2 weeks.

Detergent powders is a particular consumer product where whiteness is an important parameter, because whiteness is associated with cleaning performance. Thus, in another aspect, the invention also provides a powder detergent comprising a surfactant and a builder, and any of the particles of the invention.

The protein particles typically have a (weight/volume average) diameter of 20-3000 µm, preferably 50-2000 µm, 100-1500 µm or 250-1200 µm. In a particularly preferred embodiment, the (weight/volume average) diameter of the protein particles is 200-700 µm. The particles may be (roughly) spherical.

In an embodiment, the protein particles contain (essentially) no surfactant or bleaching agent.

### Particles/cores

The particles, or the core as referred to above, may include additional materials such as fillers, fibre materials (cellulose or synthetic fibres), stabilizing agents, solubilising agents, suspension agents, viscosity regulating agents, light spheres, plasticizers, salts, lubricants and fragrances. Both the particles and core are hereafter simply referred to as "core".

The core may include binders, such as synthetic polymer, wax, fat, or carbohydrate.

The core may comprise a salt of a multivalent cation, a reducing agent, an antioxidant, a peroxide decomposing catalyst and/or an acidic buffer component, typically as a homogenous blend; or other combinations of active and inactive ingredients.

The core may consist of an inert particle with the enzyme applied onto the surface of the inert particle, e.g., via seeded mixer granulation or layered granulation in a fluid bed. Such inert particles can be an organic particulate compound e.g. a natural compound such as agglomerated carbohydrates, e.g. sugars, starch, dextrins, flour (e.g. vegetable flour), or nonpareils. Nonpareils are spherical particles made of a seed crystal that has been built onto and rounded into a spherical shape. Nonpareils are typically made from a combination of a sugar such as sucrose, and a powder such as cornstarch. The inert particle can also be a sodium chloride or sodium sulfate crystal (or agglomerated crystals), also referred to as a seed, or other inorganic salt crystal; or a sucrose crystal.

### Preparation of core

The core can be prepared by granulating a blend of the ingredients, e.g., by a method comprising granulation techniques such as crystallization, precipitation, pan-coating, fluid bed coating, fluid bed agglomeration, rotary atomization, extrusion, prilling, spheronization, size reduction methods, drum granulation, and/or high shear granulation.

Cores without enzyme are prepared by the same techniques, but without enzyme.

Methods for preparing the core can be found in Handbook of Powder Technology; Particle size enlargement by C. E. Capes; Volume 1; 1980; Elsevier. Preparation methods include known feed and particle formulation technologies, e.g.:
a) Spray dried products, wherein a liquid enzyme-containing solution is atomized in a spray drying tower to form small droplets which during their way down the drying tower dry to form an enzyme-containing particulate material. Very small particles can be produced this way (Michael S. Showell (editor); Powdered detergents; Surfactant Science Series; 1998; vol. 71; page 140-142; Marcel Dekker).
b) Layered products, wherein the enzyme is coated as a layer around a pre-formed inert core particle, wherein an enzyme-containing solution is atomized, typically in a fluid bed apparatus wherein the pre-formed core particles are fluidized, and the enzyme-containing solution adheres to the core particles and dries up to leave a layer of dry enzyme on the surface of the core particle. Particles of a desired size can be obtained this way if a useful core particle of the desired size can be found. This type of product is described in, *e.g*., WO 97/23606
c) Absorbed core particles, wherein rather than coating the enzyme as a layer around the core, the enzyme is absorbed onto and/or into the surface of the core. Such a process is described in WO 97/39116.
d) Extrusion or pelletized products, wherein an enzyme-containing paste is pressed to pellets or under pressure is extruded through a small opening and cut into particles which are subsequently dried. Such particles usually have a considerable size because of the material in which the extrusion opening is made (usually a plate with bore holes) sets a limit on the allowable pressure drop over the extrusion opening. Also, very high extrusion pressures when using a small opening increase heat generation in the enzyme paste, which is harmful to the enzyme (see also Michael S. Showell (editor); Powdered detergents; Surfactant Science Series; 1998; vol. 71; page 140-142; Marcel Dekker).
e) Prilled products, wherein an enzyme-containing powder is suspended in molten wax and the suspension is sprayed, e.g., through a rotating disk atomiser, into a cooling chamber where the droplets quickly solidify (Michael S. Showell (editor); Powdered detergents; Surfactant Science Series; 1998; vol. 71; page 140-142; Marcel Dekker). The product obtained is one wherein the enzyme is uniformly distributed throughout an inert material instead of being concentrated on its surface. Also, US 4,016,040 and US 4,713,245 are documents relating to this technique
f) Mixer granulation products, wherein a liquid is added to a dry powder composition of, *e.g*., conventional granulating components, the enzyme being introduced either via the liquid or the powder or both. The liquid and the powder are mixed and as the moisture of the liquid is absorbed in the dry powder, the components of the dry powder will start to adhere and agglomerate and particles will build up, forming granulates comprising the enzyme. Such a process is described in US 4,106,991 and related documents EP 170360, EP 304332, EP 304331, WO 90/09440 and WO 90/09428. In a particular product of this process wherein various high-shear mixers can be used as granulators, granulates consisting of enzyme as enzyme, fillers and binders etc. are mixed with cellulose fibres to reinforce the particles to give the so-called T-granulate. Reinforced particles, being more robust, release less enzymatic dust.
g) Size reduction, wherein the cores are produced by milling or crushing of larger particles, pellets, tablets, briquettes etc. containing the enzyme. The wanted core particle fraction is obtained by sieving the milled or crushed product. Over and undersized particles can be recycled. Size reduction is described in (Martin Rhodes (editor); Principles of Powder Technology; 1990; Chapter 10; John Wiley & Sons).
h) Fluid bed granulation. Fluid bed granulation involves suspending particulates in an air stream and spraying a liquid onto the fluidized particles via nozzles. Particles hit by spray droplets get wetted and become tacky. The tacky particles collide with other particles and adhere to them and form a particle.
i) The cores may be subjected to drying, such as in a fluid bed drier. Other known methods for drying particles in the feed or detergent industry can be used by the skilled person. The drying preferably takes place at a product temperature of from 25 to 90°C. For some enzymes it is important the cores comprising the enzyme contain a low amount of water before coating. If water sensitive enzymes are coated before excessive water is removed, the excessive water will be trapped within the core and it may affect the activity of the enzyme negatively. After drying, the cores preferably contain 0.1-10 % w/w water.

### Coating(s)

The protein/enzyme particles may comprise at least one coating. Coating(s) may also be applied to the cores to improve the protein storage stability, to reduce protein dust formation during handling, to improve adherence of a protein coating onto the core, or for coloring the particles.

The coating(s) may include a salt coating, and/or a polymer coating. A polymer coating comprises polyethylene glycol (PEG), methyl hydroxy-propyl cellulose (MHPC), or polyvinyl alcohol (PVA).

Examples of enzyme particles with multiple coatings are shown in WO 93/07263 and WO 97/23606. The coating(s) may also include functional ingredients, such bleach catalysts (e.g. manganese bleach catalysts; MnTACN) and/or bleach activators (e.g. TAED, NOBS).

The coating may be applied in an amount of at least 0.1% by weight of the core, e.g., at least 0.5%, 1% or 5%. The amount may be at most 100%, 70%, 50%, 40% or 30%.

The coating is preferably at least 0.1 µm thick, particularly at least 0.5 µm, at least 1 µm or at least 5 µm. In a particular embodiment the thickness of the coating is below 100 µm. In a more particular embodiment, the thickness of the coating is below 60 µm. In an even more particular embodiment, the total thickness of the coating is below 40 µm.

The coating should encapsulate the core (and the matrix layer) by forming a substantially continuous layer. A substantially continuous layer is to be understood as a coating having few or no holes, so that the core unit it is encapsulating/enclosing has few or no uncoated areas. The layer or coating should in particular be homogeneous in thickness.

The coating can further contain other materials as known in the art, *e.g*., fillers, antisticking agents, pigments, dyes, plasticizers and/or binders, kaolin, calcium carbonate or talc.

### Salt coating

A salt coating may comprise at least 60% by weight w/w of a salt, *e.g*., at least 65%, at least 70%, at least 75%, at least 80%, at least 85%, at least 90%, at least 95% or at least 99% by weight w/w.

The salt may be added from a salt solution where the salt is completely dissolved or from a salt suspension wherein the fine particles is less than 50 µm, such as less than 10 µm or less than 5 µm.

The salt coating may comprise a single salt or a mixture of two or more salts. The salt may be water soluble, in particular having a solubility at least 0.1 grams in 100 g of water at 20°C, preferably at least 0.5 g per 100 g water, *e.g.,* at least 1 g per 100 g water, *e.g.,* at least 5 g per 100 g water.

The salt may be an inorganic salt, e.g., salts of sulfate, sulfite, phosphate, phosphonate, nitrate, chloride or carbonate or salts of simple organic acids (less than 10 carbon atoms, *e.g*., 6 or less carbon atoms) such as citrate, malonate or acetate. Examples of cations in these salts are alkali or earth alkali metal ions, the ammonium ion or metal ions of the first transition series, such as sodium, potassium, magnesium, calcium, zinc or aluminium. Examples of anions include chloride, bromide, iodide, sulfate, sulfite, bisulfite, thiosulfate, phosphate, monobasic phosphate, dibasic phosphate, hypophosphite, dihydrogen pyrophosphate, tetraborate, borate, carbonate, bicarbonate, metasilicate, citrate, malate, maleate, malonate, succinate, lactate, formate, acetate, butyrate, propionate, benzoate, tartrate, ascorbate or gluconate. In particular alkali- or earth alkali metal salts of sulfate, sulfite, phosphate, phosphonate, nitrate, chloride or carbonate or salts of simple organic acids such as citrate, malonate or acetate may be used.

The salt in the coating may have a constant relative humidity at 20°C (also referred to as 'humidity fixed point') above 60%, particularly above 70%, above 80% or above 85%, or it may be another hydrate form of such a salt (*e.g*., anhydrate). The salt coating may be as described in WO 00/01793 or WO 2006/034710.

Specific examples of suitable salts are NaCl (CH_{20°C}=76%), Na₂CO₃ (CH_{20°C}=92%), NaNO₃ (CH_{20°C}=73%), Na₂HPO₄ (CH_{20°C}=95%), Na₃PO₄ (CH_{25°C}=92%), NH₄Cl (CH_{20°C}= 79.5%), (NH₄)₂HPO₄ (CH_{20°C}= 93.0%), NH₄H₂PO₄ (CH_{20°C}= 93.1%), (NH₄)₂SO₄ (CH_{20°C}=81.1%), KCl (CH_{20°C}=85%), K₂HPO₄ (CH_{20°C}=92%), KH₂PO₄ (CH_{20°C}=96.5%), KNO₃ (CH_{20°C}=93.5%), Na₂SO4 (CH_{20°C}=93%), K₂SO₄ (CH_{20°C}=98%), KHSO₄ (CH_{20°C}=86%), MgSO₄ (CH_{20°C}=90%), ZnSO₄ (CH_{20°C}=90%) and sodium citrate (CH_{25°C}=86%). Other examples include NaH₂PO₄, (NH₄)H₂PO4, CuSO₄, Mg(NO₃)₂ and magnesium acetate.

The salt may be in anhydrous form, or it may be a hydrated salt, i.e. a crystalline salt hydrate with bound water(s) of crystallization, such as described in WO 99/32595. Specific examples include anhydrous sodium sulfate (Na₂SO₄), anhydrous magnesium sulfate (MgSO₄), magnesium sulfate heptahydrate (MgSO₄ · 7H₂O), zinc sulfate heptahydrate (ZnSO₄ · 7H₂O), sodium phosphate dibasic heptahydrate (Na₂HPO₄ · 7H₂O), magnesium nitrate hexahydrate (Mg(NO₃)₂(6H₂O)), sodium citrate dihydrate and magnesium acetate tetrahydrate.

Preferably the salt is applied as a solution of the salt, *e.g*., using a fluid bed.

The protein/enzyme particles may be essentially free of titanium dioxide. The term "essentially free of" means that the enzyme particles do not contain titanium dioxide in an amount that materially alter, or have any material effect on, the enzyme particles. In an embodiment, "essentially free of" means that the enzyme particles contain less than 0.5% w/w titanium dioxide, preferably less than 0.1% w/w titanium dioxide.

### Proteins

The protein(s) comprised in the particles of the invention may be any protein, but in particular proteins produced by fermentation.

Proteins are small (peptides; <50 amino acids) and large (polypeptides; >50 amino acids) biomolecules that perform a vast array of functions within living organisms, including catalyzing reactions, DNA replication, responding to stimuli, providing structure to cells and organisms, and transporting molecules from one location to another. Proteins are composed of chains of polymerized amino acids, which are folded in a very specific three-dimensional structure. The three-dimensional structure is critical for maintaining the function of the protein. Some chemicals can change the folding, or even unfold (denaturing) the three-dimensional structure, which will result in loss of function, such as loss of enzymatic activity.

In an embodiment, the proteins are polypeptides.

Proteins fall into at least three distinct groups, namely enzymes, cell signaling and ligand binding proteins, and structural proteins.

Enzymes are described below. Cell signaling and ligand binding proteins include many pharmaceutical proteins such as, for example, receptors, membrane proteins, ion channels, antibodies (e.g. single-domain antibodies) and hormones; while structural proteins provide stiffness and rigidity to otherwise-fluid biological components.

Preferably, the proteins are enzymes or cell signaling and ligand binding proteins; more preferably the proteins are enzymes.

### Enzymes

The enzymes used in the compositions of the invention are catalytic proteins, and the term "active enzyme protein" is defined herein as the amount of catalytic protein(s), which exhibits enzymatic activity. This can be determined using an activity based analytical enzyme assay. In such assays, the enzyme typically catalyzes a reaction generating a colored compound. The amount of the colored compound can be measured and correlated to the concentration of the active enzyme protein. This technique is well-known in the art.

The enzyme(s) may be one or more (detergent) enzymes, such as selected from the group consisting of protease, lipase, cutinase, amylase, carbohydrase, cellulase, pectinase, mannanase, arabinase, galactanase, xylanase, nuclease (DNase, RNase), dispersin, catalase, perhydrolase, and oxidase (such as laccase and/or peroxidase). More preferred detergent enzymes are selected from the group consisting of protease, lipase, amylase, cellulase, pectinase, mannanase, xylanase, nuclease (DNase, RNase), dispersin, catalase, and perhydrolase.

The enzyme may be a naturally occurring enzyme of bacterial or fungal origin, or it may be a variant derived from one or more naturally occurring enzymes by gene shuffling and/or by substituting, deleting or inserting one or more amino acids. Chemically modified or protein engineered mutants are included.

The enzyme particles, as used in the invention, contain at least one enzyme in an amount of 0.1-25% w/w active enzyme protein; preferably in an amount of 0.5-25% w/w active enzyme protein; and more preferably in an amount of 0.5-20% w/w active enzyme protein.

### Proteases

Suitable proteases may be of any origin, but are preferably of bacterial or fungal origin, optionally in the form of protein engineered or chemically modified mutants. The protease may be an alkaline protease, such as a serine protease or a metalloprotease. A serine protease may for example be of the S1 family, such as trypsin, or the S8 family such as a subtilisin. A metalloprotease may for example be a thermolysin, e.g. from the M4 family, or another metalloprotease such as those from the M5, M7 or M8 families.

The term "subtilases" refers to a sub-group of serine proteases according to Siezen et al., Protein Eng. 4 (1991) 719-737 and Siezen et al., Protein Sci. 6 (1997) 501-523. Serine proteases are a subgroup of proteases characterized by having a serine in the active site, which forms a covalent adduct with the substrate. The subtilases may be divided into six subdivisions, the Subtilisin family, the Thermitase family, the Proteinase K family, the Lantibiotic peptidase family, the Kexin family and the Pyrolysin family.

Although proteases suitable for detergent use may be obtained from a variety of organisms, including fungi such as *Aspergillus,* detergent proteases have generally been obtained from bacteria and in particular from *Bacillus.* Examples of *Bacillus* species from which subtilases have been derived include *Bacillus lentus, Bacillus alkalophilus, Bacillus subtilis, Bacillus amyloliquefaciens, Bacillus licheniformis, Bacillus pumilus* and *Bacillus gibsonii.* Particular subtilisins include *subtilisin lentus, subtilisin* Novo, *subtilisin* Carlsberg, *subtilisin* BPN', *subtilisin* 309, *subtilisin* 147 and *subtilisin* 168 and e.g. protease PD138 (described in WO 93/18140). Other useful proteases are e.g. those described in WO 01/16285 and WO 02/16547.

Examples of trypsin-like proteases include the *Fusarium* protease described in WO 94/25583 and WO 2005/040372, and the chymotrypsin proteases derived from *Cellumonas* described in WO 2005/052161 and WO 2005/052146.

Examples of metalloproteases include the neutral metalloproteases described in WO 2007/044993 such as those derived from *Bacillus amyloliquefaciens,* as well as e.g. the metalloproteases described in WO 2015/158723 and WO 2016/075078.

Examples of useful proteases are the protease variants described in WO 89/06279 WO 92/19729, WO 96/34946, WO 98/20115, WO 98/20116, WO 99/11768, WO 01/44452, WO 03/006602, WO 2004/003186, WO 2004/041979, WO 2007/006305, WO 2011/036263, WO 2014/207227, WO 2016/087617 and WO 2016/174234. Preferred protease variants may, for example, comprise one or more of the mutations selected from the group consisting of: S3T, V4I, S9R, S9E, A15T, S24G, S24R, K27R, N42R, S55P, G59E, G59D, N60D, N60E, V66A, N74D, S85R, A96S, S97G, S97D, S97A, S97SD, S99E, S99D, S99G, S99M, S99N, S99R, S99H, S101A, V102I, V102Y, V102N, S104A, G116V, G116R, H118D, H118N, A120S, S126L, P127Q, S128A, S154D, A156E, G157D, G157P, S158E, Y161A, R164S, Q176E, N179E, S182E, Q185N, A188P, G189E, V193M, N198D, V199I, Q200L, Y203W, S206G, L211Q, L211D, N212D, N212S, M216S, A226V, K229L, Q230H, Q239R, N246K, S253D, N255W, N255D, N255E, L256E, L256D T268A and R269H, wherein position numbers correspond to positions of the *Bacillus lentus* protease shown in SEQ ID NO: 1 of WO 2016/001449. Protease variants having one or more of these mutations are preferably variants of the *Bacillus lentus* protease (Savinase^{®}, also known as subtilisin 309) shown in SEQ ID NO: 1 of WO 2016/001449 or of the *Bacillus amyloliquefaciens* protease (BPN') shown in SEQ ID NO: 2 of WO 2016/001449. Such protease variants preferably have at least 80% sequence identity to SEQ ID NO: 1 or to SEQ ID NO: 2 of WO 2016/001449.

Another protease of interest is the alkaline protease from *Bacillus lentus* DSM 5483, as described for example in WO 91/02792, and variants thereof which are described for example in WO 92/21760, WO 95/23221, EP 1921147, EP 1921148 and WO 2016/096711.

The protease may alternatively be a variant of the TY145 protease having SEQ ID NO: 1 of WO 2004/067737, for example a variant comprising a substitution at one or more positions corresponding to positions 27, 109, 111, 171, 173, 174, 175, 180, 182, 184, 198, 199 and 297 of SEQ ID NO: 1 of WO 2004/067737, wherein said protease variant has a sequence identity of at least 75% but less than 100% to SEQ ID NO: 1 of WO 2004/067737. TY145 variants of interest are described in e.g. WO 2015/014790, WO 2015/014803, WO 2015/014804, WO 2016/097350, WO 2016/097352, WO 2016/097357 and WO 2016/097354.

Examples of preferred proteases include:
(a) variants of SEQ ID NO: 1 of WO 2016/001449 comprising two or more substitutions selected from the group consisting of S9E, N43R, N76D, Q206L, Y209W, S259D and L262E, for example a variant with the substitutions S9E, N43R, N76D, V205I, Q206L, Y209W, S259D, N261W and L262E, or with the substitutions S9E, N43R, N76D, N185E, S188E, Q191N, A194P, Q206L, Y209W, S259D and L262E, wherein position numbers are based on the numbering of SEQ ID NO: 2 of WO 2016/001449;
(b) a variant of the polypeptide of SEQ ID NO: 1 of WO 2016/001449 with the mutation S99SE, wherein position numbers are based on the numbering of SEQ ID NO: 2 of WO 2016/001449;
(c) a variant of the polypeptide of SEQ ID NO: 1 of WO 2016/001449 with the mutation S99AD, wherein position numbers are based on the numbering of SEQ ID NO: 2 of WO 2016/001449;
(d) a variant of the polypeptide of SEQ ID NO: 1 of WO 2016/001449 with the substitutions Y167A+R170S+A194P, wherein position numbers are based on the numbering of SEQ ID NO: 2 of WO 2016/001449;
(e) a variant of the polypeptide of SEQ ID NO: 1 of WO 2016/001449 with the substitutions S9R+A15T+V68A+N218D+Q245R, wherein position numbers are based on the numbering of SEQ ID NO: 2 of WO 2016/001449;
(f) a variant of the polypeptide of SEQ ID NO: 1 of WO 2016/001449 with the substitutions S9R+A15T+G61E+V68A+A194P+V205I+Q245R+N261D, wherein position numbers are based on the numbering of SEQ ID NO: 2 of WO 2016/001449;
(g) a variant of the polypeptide of SEQ ID NO: 1 of WO 2016/001449 with the substitutions S99D+S101R/E+S103A+V104I+G160S; for example a variant of SEQ ID NO: 1 of WO 2016/001449 with the substitutions S3T+V4I+S99D+S101E+S103A+V104I+G160S+V205I, wherein position numbers are based on the numbering of SEQ ID NO: 2 of WO 2016/001449;
(h) a variant of the polypeptide of SEQ ID NO: 2 of WO 2016/001449 with the substitutions S24G+S53G+S78N+S101N+G128A/S+Y217Q, wherein position numbers are based on the numbering of SEQ ID NO: 2 of WO 2016/001449;
(i) the polypeptide disclosed in GENESEQP under accession number BER84782, corresponding to SEQ ID NO: 302 in WO 2017/210295;
(j) a variant of the polypeptide of SEQ ID NO: 1 of WO 2016/001449 with the substitutions S99D+S101E+S103A+V104I+S156D+G160S+L262E, wherein position numbers are based on the numbering of SEQ ID NO: 2 of WO 2016/001449;
(k) a variant of the polypeptide of SEQ ID NO: 1 of WO 2016/001449 with the substitutions S9R+A15T+G61E+V68A+N76D+S99G+N218D+Q245R, wherein position numbers are based on the numbering of SEQ ID NO: 2 of WO 2016/001449;
(l) a variant of the polypeptide of SEQ ID NO: 1 of WO 2016/001449 with the substitutions V68A+S106A, wherein position numbers are based on the numbering of SEQ ID NO: 2 of WO 2016/001449; and
(m) a variant of the polypeptide of SEQ ID NO: 1 of WO 2004/067737 with the substitutions S27K+N109K+S111E+S171E+S173P+G174K+S175P+F180Y+G182A+L184F+ Q198E+N199+T297P, wherein position numbers are based on the numbering of SEQ ID NO: 1 of WO 2004/067737.

Suitable commercially available protease enzymes include those sold under the trade names Alcalase^{®}, Duralase^{™}, Durazym^{™}, Relase^{®}, Relase^{®} Ultra, Savinase^{®}, Savinase^{®} Ultra, Primase^{™}, Polarzyme^{®}, Kannase^{®}, Liquanase^{®}, Liquanase^{®} Ultra, Ovozyme^{®}, Coronase^{®}, Coronase^{®} Ultra, Blaze^{®}, Blaze Evity^{®} 100T, Blaze Evity^{®} 125T, Blaze Evity^{®} 150T, Blaze Evity^{®} 200T, Neutrase^{®}, Everlase^{®}, Esperase^{®}, Progress^{®} Uno, Progress^{®} In and Progress^{®} Excel (Novozymes A/S), those sold under the tradename Maxatase^{™}, Maxacal^{™}, Maxapem^{®}, Purafect^{®} Ox, Purafect^{®} OxP, Puramax^{®}, FN2^{™}, FN3^{™}, FN4^{ex™}, Excellase^{®}, Excellenz^{™} P1000, Excellenz^{™} P1250, Eraser^{™}, Preferenz^{®} P100, Purafect Prime, Preferenz P110^{™}, Effectenz P1000^{™}, Purafect^{®}, Effectenz P1050^{™}, Purafect^{®} Ox, Effectenz^{™} P2000, Purafast^{™}, Properase^{®}, Opticlean^{™} and Optimase^{®} (Danisco/DuPont), BLAP (sequence shown in Figure 29 of US 5352604) and variants hereof (Henkel AG), and KAP (*Bacillus alkalophilus* subtilisin) from Kao.

### Lipases and Cutinases

Suitable lipases and cutinases include those of bacterial or fungal origin. Chemically modified or protein engineered mutant enzymes are included. Examples include lipase from *Thermomyces,* e.g. from *T. lanuginosus* (previously named *Humicola lanuginosa*) as described in EP258068 and EP305216, cutinase from *Humicola,* e.g. *H. insolens* (WO96/13580), lipase from strains of *Pseudomonas* (some of these now renamed to *Burkholderia*), e.g. *P. alcaligenes* or *P. pseudoalcaligenes* (EP218272), *P. cepacia* (EP331376), *P. sp.* strain SD705 (WO95/06720 & WO96/27002), *P. wisconsinensis* (WO96/12012), GDSL-type *Streptomyces* lipases (WO10/065455), cutinase from *Magnaporthe grisea* (WO10/107560), cutinase from *Pseudomonas mendocina* (US5,389,536), lipase from *Thermobifida fusca* (WO11/084412), *Geobacillus stearothermophilus* lipase (WO11/084417), lipase from *Bacillus subtilis* (WO11/084599), and lipase from *Streptomyces griseus* (WO11/150157) and *S. pristinaespiralis* (WO12/137147).

Other examples are lipase variants such as those described in EP407225, WO92/05249, WO94/01541, WO94/25578, WO95/14783, WO95/30744, WO95/35381, WO95/22615, WO96/00292, WO97/04079, WO97/07202, WO00/34450, WO00/60063, WO01/92502, WO07/87508 and WO09/109500.

Preferred commercial lipase products include Lipolase^{™}, Lipex^{™}, Lipolex^{™} and Lipoclean^{™} (Novozymes A/S), Lumafast (originally from Genencor) and Lipomax (originally from Gist-Brocades).

Still other examples are lipases sometimes referred to as acyltransferases or perhydrolases, e.g. acyltransferases with homology to *Candida antarctica* lipase A (WO10/111143), acyltransferase from *Mycobacterium smegmatis* (WO05/56782), perhydrolases from the CE 7 family (WO09/67279), and variants of the *M. smegmatis* perhydrolase in particular the S54V variant used in the commercial product Gentle Power Bleach from Huntsman Textile Effects Pte Ltd (WO10/100028).

### Amylases

Suitable amylases may be an alpha-amylase or a glucoamylase and may be of bacterial or fungal origin. Chemically modified or protein engineered mutants are included. Amylases include, for example, alpha-amylases obtained from *Bacillus, e.g.,* a special strain of *Bacillus licheniformis,* described in more detail in GB 1,296,839.

Suitable amylases include amylases having SEQ ID NO: 2 in WO 95/10603 or variants having 90% sequence identity to SEQ ID NO: 3 thereof. Preferred variants are described in WO 94/02597, WO 94/18314, WO 97/43424 and SEQ ID NO: 4 of WO 99/019467, such as variants with substitutions in one or more of the following positions: 15, 23, 105, 106, 124, 128, 133, 154, 156, 178, 179, 181, 188, 190, 197, 201, 202, 207, 208, 209, 211, 243, 264, 304, 305, 391, 408, and 444.

Different suitable amylases include amylases having SEQ ID NO: 6 in WO 02/010355 or variants thereof having 90% sequence identity to SEQ ID NO: 6. Preferred variants of SEQ ID NO: 6 are those having a deletion in positions 181 and 182 and a substitution in position 193.

Other amylases which are suitable are hybrid alpha-amylase comprising residues 1-33 of the alpha-amylase derived from *B. amyloliquefaciens* shown in SEQ ID NO: 6 of WO 2006/066594 and residues 36-483 of the *B. licheniformis* alpha-amylase shown in SEQ ID NO: 4 of WO 2006/066594 or variants having 90% sequence identity thereof. Preferred variants of this hybrid alpha-amylase are those having a substitution, a deletion or an insertion in one of more of the following positions: G48, T49, G107, H156, A181, N190, M197, 1201, A209 and Q264. Most preferred variants of the hybrid alpha-amylase comprising residues 1-33 of the alpha-amylase derived from *B. amyloliquefaciens* shown in SEQ ID NO: 6 of WO 2006/066594 and residues 36-483 of SEQ ID NO: 4 are those having the substitutions:
M197T;
H156Y+A181T+N190F+A209V+Q264S; or
G48A+T49I+G107A+H156Y+A181T+N190F+1201F+A209V+Q264S.

Further amylases which are suitable are amylases having SEQ ID NO: 6 in WO 99/019467 or variants thereof having 90% sequence identity to SEQ ID NO: 6. Preferred variants of SEQ ID NO: 6 are those having a substitution, a deletion or an insertion in one or more of the following positions: R181, G182, H183, G184, N195, I206, E212, E216 and K269. Particularly preferred amylases are those having deletion in positions R181 and G182, or positions H183 and G184.

Additional amylases which can be used are those having SEQ ID NO: 1, SEQ ID NO: 3, SEQ ID NO: 2 or SEQ ID NO: 7 of WO 96/023873 or variants thereof having 90% sequence identity to SEQ ID NO: 1, SEQ ID NO: 2, SEQ ID NO: 3 or SEQ ID NO: 7. Preferred variants of SEQ ID NO: 1, SEQ ID NO: 2, SEQ ID NO: 3 or SEQ ID NO: 7 are those having a substitution, a deletion or an insertion in one or more of the following positions: 140, 181, 182, 183, 184, 195, 206, 212, 243, 260, 269, 304 and 476, using SEQ ID 2 of WO 96/023873 for numbering. More preferred variants are those having a deletion in two positions selected from 181, 182, 183 and 184, such as 181 and 182, 182 and 183, or positions 183 and 184. Most preferred amylase variants of SEQ ID NO: 1, SEQ ID NO: 2 or SEQ ID NO: 7 are those having a deletion in positions 183 and 184 and a substitution in one or more of positions 140, 195, 206, 243, 260, 304 and 476.

Other amylases which can be used are amylases having SEQ ID NO: 2 of WO 08/153815, SEQ ID NO: 10 in WO 01/66712 or variants thereof having 90% sequence identity to SEQ ID NO: 2 of WO 08/153815 or 90% sequence identity to SEQ ID NO: 10 in WO 01/66712. Preferred variants of SEQ ID NO: 10 in WO 01/66712 are those having a substitution, a deletion or an insertion in one of more of the following positions: 176, 177, 178, 179, 190, 201, 207, 211 and 264.

Further suitable amylases are amylases having SEQ ID NO: 2 of WO 09/061380 or variants having 90% sequence identity to SEQ ID NO: 2 thereof. Preferred variants of SEQ ID NO: 2 are those having a truncation of the C-terminus and/or a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred variants of SEQ ID NO: 2 are those having the substitution in one of more of the following positions: Q87E,R, Q98R, S125A, N128C, T131I, T165I, K178L, T182G, M201L, F202Y, N225E,R, N272E,R, S243Q,A,E,D, Y305R, R309A, Q320R, Q359E, K444E and G475K and/or deletion in position R180 and/or S181 or of T182 and/or G183. Most preferred amylase variants of SEQ ID NO: 2 are those having the substitutions:
N128C+K178L+T182G+Y305R+G475K;
N128C+K178L+T182G+F202Y+Y305R+D319T+G475K;
S125A+N128C+K178L+T182G+Y305R+G475K; or
S125A+N128C+T131I+T165I+K178L+T182G+Y305R+G475K wherein the variants are C-terminally truncated and optionally further comprises a substitution at position 243 and/or a deletion at position 180 and/or position 181.

Further suitable amylases are amylases having SEQ ID NO: 1 of WO13184577 or variants having 90% sequence identity to SEQ ID NO: 1 thereof. Preferred variants of SEQ ID NO: 1 are those having a substitution, a deletion or an insertion in one of more of the following positions: K176, R178, G179, T180, G181, E187, N192, M199, I203, S241, R458, T459, D460, G476 and G477. More preferred variants of SEQ ID NO: 1 are those having the substitution in one of more of the following positions: K176L, E187P, N192FYH, M199L, I203YF, S241QADN, R458N, T459S, D460T, G476K and G477K and/or deletion in position R178 and/or S179 or of T180 and/or G181. Most preferred amylase variants of SEQ ID NO: 1 are those having the substitutions:
E187P+I203Y+G476K
E187P+I203Y+R458N+T459S+D460T+G476K
wherein the variants optionally further comprises a substitution at position 241 and/or a deletion at position 178 and/or position 179.

Further suitable amylases are amylases having SEQ ID NO: 1 of WO10104675 or variants having 90% sequence identity to SEQ ID NO: 1 thereof. Preferred variants of SEQ ID NO: 1 are those having a substitution, a deletion or an insertion in one of more of the following positions: N21, D97, V128 K177, R179, S180, 1181, G182, M200, L204, E242, G477 and G478. More preferred variants of SEQ ID NO: 1 are those having the substitution in one of more of the following positions: N21D, D97N, V128I K177L, M200L, L204YF, E242QA, G477K and G478K and/or deletion in position R179 and/or S180 or of 1181 and/or G182. Most preferred amylase variants of SEQ ID NO: 1 are those having the substitutions:
N21D+D97N+V128I
wherein the variants optionally further comprise a substitution at position 200 and/or a deletion at position 180 and/or position 181.

Other suitable amylases are the alpha-amylase having SEQ ID NO: 12 in WO01/66712 or a variant having at least 90% sequence identity to SEQ ID NO: 12. Preferred amylase variants are those having a substitution, a deletion or an insertion in one of more of the following positions of SEQ ID NO: 12 in WO01/66712: R28, R118, N174; R181, G182, D183, G184, G186, W189, N195, M202, Y298, N299, K302, S303, N306, R310, N314; R320, H324, E345, Y396, R400, W439, R444, N445, K446, Q449, R458, N471, N484. Particular preferred amylases include variants having a deletion of D183 and G184 and having the substitutions R118K, N195F, R320K and R458K, and a variant additionally having substitutions in one or more position selected from the group: M9, G149, G182, G186, M202, T257, Y295, N299, M323, E345 and A339, most preferred a variant that additionally has substitutions in all these positions.

Other examples are amylase variants such as those described in WO2011/098531, WO2013/001078 and WO2013/001087.

Commercially available amylases are Duramyl^{™}, Termamyl^{™}, Fungamyl^{™}, Stainzyme^{™}, Stainzyme Plus^{™}, Natalase^{™}, Liquozyme X and BAN^{™} (from Novozymes A/S), and Rapidase^{™}, Purastar^{™}/Effectenz^{™}, Powerase, Preferenz S1000, Preferenz S100 and Preferenz S110 (from Genencor International Inc./DuPont).

### Cellulases

Suitable cellulases include mono-component and mixtures of enzymes of bacterial or fungal origin. Chemically modified or protein engineered mutants are also contemplated. The cellulase may for example be a mono-component or a mixture of mono-component endo-1,4-beta-glucanase also referred to as endoglucanase.

Suitable cellulases include those from the genera *Bacillus, Pseudomonas, Humicola, Myceliophthora, Fusarium, Thielavia, Trichoderma,* and *Acremonium.* Exemplary cellulases include a fungal cellulase from *Humicola insolens* (US 4,435,307) or from *Trichoderma,* e.g. *T. reesei* or *T. viride.* Other suitable cellulases are from *Thielavia e.g. Thielavia terrestris* as described in WO 96/29397 or the fungal cellulases produced from *Myceliophthora thermophila* and *Fusarium oxysporum* disclosed in US 5,648,263, US 5,691,178, US 5,776,757, WO 89/09259 and WO 91/17244. Also relevant are cellulases from *Bacillus* as described in WO 02/099091 and JP 2000210081. Suitable cellulases are alkaline or neutral cellulases having care benefits. Examples of cellulases are described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307.

Other cellulases are endo-beta-1,4-glucanase enzyme having a sequence of at least 97% identity to the amino acid sequence of position 1 to position 773 of SEQ ID NO:2 of WO 2002/099091 or a family 44 xyloglucanase, which a xyloglucanase enzyme having a sequence of at least 60% identity to positions 40-559 of SEQ ID NO: 2 of WO 2001/062903.

Commercially available cellulases include Carezyme^{®}, Carezyme^{®} Premium, Celluzyme^{®}, Celluclean^{®}, Celluclast^{®}, Endolase^{®}, Renozyme^{®}; Whitezyme^{®} Celluclean^{®} Classic, Cellusoft^{®} (Novozymes A/S), Puradax^{®}, Puradax HA, and Puradax EG (available from Genencor International Inc.) and KAC-500(B)^{™} (Kao Corporation).

### Mannanases

Suitable mannanases include those of bacterial or fungal origin. Chemically or genetically modified mutants are included. The mannanase may be an alkaline mannanase of Family 5 or 26. It may be a wild-type from *Bacillus* or *Humicola,* particularly *B. agaradhaerens, B. licheniformis, B. halodurans, B. clausii,* or *H. insolens.* Suitable mannanases are described in WO 1999/064619. A commercially available mannanase is Mannaway (Novozymes A/S).

### Nucleases

Suitable nucleases include deoxyribonucleases (DNases) and ribonucleases (RNases) which are any enzyme that catalyzes the hydrolytic cleavage of phosphodiester linkages in the DNA or RNA backbone respectively, thus degrading DNA and RNA. There are two primary classifications based on the locus of activity. Exonucleases digest nucleic acids from the ends. Endonucleases act on regions in the middle of target molecules. The nuclease is preferably a DNase, which is preferable is obtainable from a microorganism, preferably a bacterium; in particular a DNase which is obtainable from a species of *Bacillus* is preferred; in particular a DNase which is obtainable from *Bacillus cibi, Bacillus subtilis* or *Bacillus licheniformis* is preferred. Examples of such DNases are described in WO 2011/098579, WO2014/087011 and WO2017/060475.

### Dispersins

Suitable dispersins are polypeptides having hexosaminidase activity, EC 3.2.1.- that catalyzes the hydrolysis of β-1,6-glycosidic linkages of N-acetyl-glucosamine polymers (poly-N-acetylglucosamine) found, *e.g*., in biofilm.

### Peroxidases/Oxidases

A suitable peroxidase is preferably a peroxidase enzyme comprised by the enzyme classification EC 1.11.1.7, as set out by the Nomenclature Committee of the International Union of Biochemistry and Molecular Biology (IUBMB), or any fragment derived therefrom, exhibiting peroxidase activity.

Suitable peroxidases include those of plant, bacterial or fungal origin. Chemically modified or protein engineered mutants are included. Examples of useful peroxidases include peroxidases from *Coprinopsis, e.g.,* from *C. cinerea* (EP 179,486), and variants thereof as those described in WO 93/24618, WO 95/10602, and WO 98/15257.

Suitable peroxidases also include a haloperoxidase enzyme, such as chloroperoxidase, bromoperoxidase and compounds exhibiting chloroperoxidase or bromoperoxidase activity. Haloperoxidases are classified according to their specificity for halide ions. Chloroperoxidases (E.C. 1.11.1.10) catalyze formation of hypochlorite from chloride ions.The haloperoxidase may be a chloroperoxidase. Preferably, the haloperoxidase is a vanadium haloperoxidase, *i.e.,* a vanadate-containing haloperoxidase. In a preferred method the vanadate-containing haloperoxidase is combined with a source of chloride ion.

Suitable oxidases include, in particular, any laccase enzyme comprised by the enzyme classification EC 1.10.3.2, or any fragment derived therefrom exhibiting laccase activity, or a compound exhibiting a similar activity, such as a catechol oxidase (EC 1.10.3.1), an o-aminophenol oxidase (EC 1.10.3.4), or a bilirubin oxidase (EC 1.3.3.5).

### Solid detergent compositions

The invention also provides solid detergent compositions comprising the particles of the invention in combination with one or more additional cleaning composition (detergent) components, as described below. The choice of additional components is within the skill of the artisan and includes conventional ingredients, including the exemplary non-limiting components set forth below. Such detergent compositions may be essentially free of titanium dioxide.

The choice of additional detergent components may include, for textile care, the consideration of the type of textile to be cleaned, the type and/or degree of soiling, the temperature at which cleaning is to take place, and the formulation of the detergent product. Although components mentioned below are categorized by general header according to a particular functionality, this is not to be construed as a limitation, as a component may comprise additional functionalities as will be appreciated by the skilled artisan.

In one embodiment, the invention is directed to an ADW (Automatic Dish Wash) compositions comprising one or more additional ADW composition components. The choice of additional components is within the skill of the artisan and includes conventional ingredients, including the exemplary non-limiting components set forth below.

The solid detergent composition may be composed essentially of biodegradable materials (be essentially biobased). In a preferred embodiment, the solid detergent composition is >95% biobased according to USDA Certified Biobased Products (biobased content measured using ASTM D6866); more preferably >97% biobased, and most preferably >99% biobased.

### Surfactants

The cleaning composition may comprise one or more surfactants, which may be anionic and/or cationic and/or non-ionic and/or semi-polar and/or zwitterionic, or a mixture thereof. In a particular embodiment, the detergent composition includes a surfactant system (comprising more than one surfactant) e.g. a mixture of one or more nonionic surfactants and one or more anionic surfactants. In one embodiment the detergent comprises at least one anionic surfactant than at least one non-ionic surfactant, the weight ratio of anionic to nonionic surfactant may be from 10:1 to 1:10. In one embodiment the amount of anionic surfactant is higher than the amount of non-ionic surfactant e.g. the weight ratio of anionic to non-ionic surfactant may be from 10:1 to 1.1:1 or from 5:1 to 1.5:1. The amount of anionic to non-ionic surfactant may also be equal and the weight ratios 1:1. In one embodiment the amount of non-ionic surfactant is higher than the amount of anionic surfactant and the weight ratio may be 1:10 to 1:1.1. Preferably the weight ratio of anionic to non-ionic surfactant is from 10:1 to 1:10, such as from 5:1 to 1:5, or from 5:1 to 1:1.2. Preferably, the weight fraction of non-ionic surfactant to anionic surfactant is from 0 to 0.5 or 0 to 0.2 thus non-ionic surfactant can be present or absent if the weight fraction is 0, but if non-ionic surfactant is present, then the weight fraction of the nonionic surfactant is preferably at most 50% or at most 20% of the total weight of anionic surfactant and non-ionic surfactant. Light duty detergent usually comprises more nonionic than anionic surfactant and there the fraction of non-ionic surfactant to anionic surfactant is preferably from 0.5 to 0.9. The total weight of surfactant(s) is typically present at a level of from about 0.1% to about 60% by weight, such as about 1% to about 40%, or about 3% to about 20%, or about 3% to about 10%. The surfactant(s) is chosen based on the desired cleaning application, and may include any conventional surfactant(s) known in the art. When included therein the detergent will usually contain from about 1% to about 40% by weight of an anionic surfactant, such as from about 5% to about 30%, including from about 5% to about 15%, or from about 15% to about 20%, or from about 20% to about 25% of an anionic surfactant. Non-limiting examples of anionic surfactants include sulfates and sulfonates, typically available as sodium or potassium salts or salts of monoethanolamine (MEA, 2-aminoethan-1-ol) or triethanolamine (TEA, 2,2',2"-nitrilotriethan-1-ol); in particular, linear alkylbenzenesulfonates (LAS), isomers of LAS such as branched alkylbenzenesulfonates (BABS) and phenylalkanesulfonates; olefin sulfonates, in particular alpha-olefinsulfonates (AOS); alkyl sulfates (AS), in particular fatty alcohol sulfates (FAS), *i.e.,* primary alcohol sulfates (PAS) such as dodecyl sulfate; alcohol ethersulfates (AES or AEOS or FES, also known as alcohol ethoxysulfates or fatty alcohol ether sulfates); paraffin sulfonates (PS) including alkane-1-sulfonates and secondary alkanesulfonates (SAS); ester sulfonates, including sulfonated fatty acid glycerol esters and alpha-sulfo fatty acid methyl esters (alpha-SFMe or SES or MES); alkyl- or alkenylsuccinic acids such as dodecenyl/tetradecenyl succinic acid (DTSA); diesters and monoesters of sulfosuccinic acid; fatty acid derivatives of amino acids. Furthermore, salts of fatty acids (soaps) may be included.

When included therein the detergent will usually contain from about 1% to about 40% by weight of a cationic surfactant, for example from about 0.5% to about 30%, in particular from about 1% to about 20%, from about 3% to about 10%, such as from about 3% to about 5%, from about 8% to about 12% or from about 10% to about 12%. Non-limiting examples of cationic surfactants include alkyldimethylethanolamine quat (ADMEAQ), cetyltrimethylammonium bromide (CTAB), dimethyldistearylammonium chloride (DSDMAC), and alkylbenzyldimethylammonium, alkyl quaternary ammonium compounds, alkoxylated quaternary ammonium (AQA) compounds, ester quats, and combinations thereof.

When included therein the detergent will usually contain from about 0.2% to about 40% by weight of a nonionic surfactant, for example from about 0.5% to about 30%, in particular from about 1% to about 20%, from about 3% to about 10%, such as from about 3% to about 5%, from about 8% to about 12%, or from about 10% to about 12%. Non-limiting examples of nonionic surfactants include alcohol ethoxylates (AE or AEO) e.g. the AEO-series such as AEO-7, alcohol propoxylates, in particular propoxylated fatty alcohols (PFA), ethoxylated and propoxylated alcohols, alkoxylated fatty acid alkyl esters, such as ethoxylated and/or propoxylated fatty acid alkyl esters (in particular methyl ester ethoxylates, MEE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxyalkyl fatty acid amides, or N-acyl N-alkyl derivatives of glucosamine (glucamides, GA, or fatty acid glucamides, FAGA), as well as products available under the trade names SPAN and TWEEN, and combinations thereof.

When included therein the detergent will usually contain from about 0.01 to about 10 % by weight of a semipolar surfactant. Non-limiting examples of semipolar surfactants include amine oxides (AO) such as alkyldimethylamine oxides, in particular N-(coco alkyl)-N,N-dimethylamine oxide and N-(tallow-alkyl)-N,N-bis(2-hydroxyethyl)amine oxide, and combinations thereof.

When included therein the detergent will usually contain from about 0.01 % to about 10 % by weight of a zwitterionic surfactant. Non-limiting examples of zwitterionic surfactants include betaines such as alkyldimethylbetaines, sulfobetaines, and combinations thereof.

Additional bio-based surfactants may be used e.g. wherein the surfactant is a sugar-based non-ionic surfactant which may be a hexyl-β-D-maltopyranoside, thiomaltopyranoside or a cyclic-maltopyranoside, such as described in EP2516606 B1.

### Builders and Co-Builders

The detergent composition may contain about 0-65% by weight, such as about 5% to about 50% of a detergent builder or co-builder, or a mixture thereof. In a dish wash detergent, the level of builder is typically in the range 40-65%, particularly in the range 50-65%. The builder and/or co-builder may particularly be a chelating agent that forms water-soluble complexes with Ca and Mg. Any builder and/or co-builder known in the art for use in cleaning detergents may be utilized.

Non-limiting examples of builders include zeolites, diphosphates (pyrophosphates), triphosphates such as sodium triphosphate (STP or STPP), carbonates such as sodium carbonate, soluble silicates such as sodium metasilicate, layered silicates (e.g., SKS-6 from Clariant), ethanolamines such as 2-aminoethan-1-ol (MEA), diethanolamine (DEA, also known as 2,2'-iminodiethan-1-ol), triethanolamine (TEA, also known as 2,2',2"-nitrilotriethan-1-ol), and (carboxymethyl)inulin (CMI), and combinations thereof.

The detergent composition may also contain from about 0-50% by weight, such as about 5% to about 30%, of a detergent co-builder. The detergent composition may include a co-builder alone, or in combination with a builder, for example a zeolite builder. Non-limiting examples of co-builders include homopolymers of polyacrylates or copolymers thereof, such as poly(acrylic acid) (PAA) or copoly(acrylic acid/maleic acid) (PAA/PMA). Further non-limiting examples include citrate, chelators such as aminocarboxylates, aminopolycarboxylates and phosphonates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-N,N'-disuccinic acid (EDDS), methylglycinediacetic acid (MGDA), glutamic acid-N,N-diacetic acid (GLDA), 1-hydroxyethane-1,1-diylbis(phosphonic acid (HEDP), ethylenediaminetetramethylenetetrakis(phosphonic acid) (EDTMPA), diethylenetriaminepentamethylenepentakis(phosphonic acid) (DTMPA or DTPMPA), N-(2-hydroxyethyl)iminodiacetic acid (EDG), aspartic acid-N-monoacetic acid (ASMA), aspartic acid-N,N-diacetic acid (ASDA), aspartic acid-N-monopropionic acid (ASMP), iminodisuccinic acid (IDA), N-(2-sulfomethyl)aspartic acid (SMAS), N-(2-sulfoethyl)aspartic acid (SEAS), N-(2-sulfomethyl)glutamic acid (SMGL), N-(2-sulfoethyl)glutamic acid (SEGL), N-methyliminodiacetic acid (MIDA), serine-N,N-diacetic acid (SEDA), isoserine-N,N-diacetic acid (ISDA), phenylalanine-N,N-diacetic acid (PHDA), anthranilic acid-N,N-diacetic acid (ANDA), sulfanilic acid-N,N-diacetic acid (SLDA), taurine-N,N-diacetic acid (TUDA) and sulfomethyl-N,N-diacetic acid (SMDA), N-(2-hydroxyethyl)ethylenediamine-N,N',N"-triacetic acid (HEDTA), diethanolglycine (DEG), aminotrimethylenetris(phosphonic acid) (ATMP), and combinations and salts thereof. Further exemplary builders and/or co-builders are described in, e.g., WO 09/102854, US 5977053.

The solid detergent composition of the invention may comprise a strong sequestering builder; such as at least 0.1% w/w, at least 0.5% w/w, or at least 1% w/w of a strong sequestering builder. Examples of strong sequestering builders are EDTA, EDTMP, NTMP, DTPMP, MGDA, NTA, HEDP, STPP, IDS, and GLDA.

Sequestering builders are different from precipitating builders in that no significant amount of precipitate is formed when the builder is used in an amount sufficient to combine with all of the calcium ions in an aqueous solution with 7°dH water hardness (German hardness) initially at neutral pH. Strong builders are classified as high efficiency chelators that can bind the divalent cations such as Ca²⁺ strongly with a logarithmic stability constant (Log K_{Ca}) of the cation/chelator complex of above 5, particularly above 6 or above 7. The stability constants are determined at an ionic strength of 0.1 M and at a temperature of 25°C.

### Bleaching Systems

The cleaning composition may contain 0-50% by weight, such as 1-40%, such as 1-30%, such as about 1% to about 20%, of a bleaching system. Any oxygen-based bleaching system comprising components known in the art for use in cleaning detergents may be utilized. Suitable bleaching system components include sources of hydrogen peroxide; peracids and sources of peracids (bleach activators); and bleach catalysts or boosters.

Suitable sources of hydrogen peroxide are inorganic persalts, including alkali metal salts such as sodium percarbonate and sodium perborates (usually mono- or tetrahydrate), and hydrogen peroxide-urea.

Peracids may be (a) incorporated directly as preformed peracids or (b) formed in situ in the wash liquor from hydrogen peroxide and a bleach activator (perhydrolysis) or (c) formed in situ in the wash liquor from hydrogen peroxide and a perhydrolase and a suitable substrate for the latter, e.g., an ester.

Suitable preformed peracids include, but are not limited to, peroxycarboxylic acids such as peroxybenzoic acid and its ring-substituted derivatives, peroxy-α-naphthoic acid, peroxyphthalic acid, peroxylauric acid, peroxystearic acid, ε-phthalimidoperoxycaproic acid [phthalimidoperoxyhexanoic acid (PAP)], and o-carboxybenzamidoperoxycaproic acid; aliphatic and aromatic diperoxydicarboxylic acids such as diperoxydodecanedioic acid, diperoxyazelaic acid, diperoxysebacic acid, diperoxybrassylic acid, 2-decyldiperoxybutanedioic acid, and diperoxyphthalic, -isophthalic and -terephthalic acids; perimidic acids; peroxymonosulfuric acid; peroxydisulfuric acid; peroxyphosphoric acid; peroxysilicic acid; and mixtures of said compounds. It is understood that the peracids mentioned may in some cases be best added as suitable salts, such as alkali metal salts (e.g., Oxone^{®}) or alkaline earth-metal salts.

Suitable bleach activators include those belonging to the class of esters, amides, imides, nitriles or anhydrides and, where applicable, salts thereof. Suitable examples are tetraacetylethylenediamine (TAED), sodium 4-[(3,5,5-trimethylhexanoyl)oxy]benzene-1-sulfonate (ISONOBS), sodium 4-(dodecanoyloxy)benzene-1-sulfonate (LOBS), sodium 4-(decanoyloxy)benzene-1-sulfonate, 4-(decanoyloxy)benzoic acid (DOBA), sodium 4-(nonanoyloxy)benzene-1-sulfonate (NOBS), and/or those disclosed in WO98/17767. A particular family of bleach activators of interest was disclosed in EP624154 and particularly preferred in that family is acetyl triethyl citrate (ATC). ATC or a short chain triglyceride like triacetin has the advantage that they are environmentally friendly. Furthermore, acetyl triethyl citrate and triacetin have good hydrolytical stability in the product upon storage and are efficient bleach activators. Finally, ATC is multifunctional, as the citrate released in the perhydrolysis reaction may function as a builder.

### Bleach catalysts and boosters

The bleaching system may also include a bleach catalyst or booster. Some non-limiting examples of bleach catalysts that may be used in the compositions of the present invention include manganese oxalate, manganese acetate, manganese-collagen, cobalt-amine catalysts and manganese triazacyclononane (MnTACN) catalysts; particularly preferred are complexes of manganese with 1,4,7-trimethyl-1,4,7-triazacyclononane (Me3-TACN) or 1,2,4,7-tetramethyl-1,4,7-triazacyclononane (Me4-TACN), in particular Me3-TACN, such as the dinuclear manganese complex [(Me3-TACN)Mn(O)3Mn(Me3-TACN)](PF6)2, and [2,2',2"-nitrilotris(ethane-1,2-diylazanylylidene-κN-methanylylidene)triphenolato-κ3O]manganese(III). The bleach catalysts may also be other metal compounds; such as iron or cobalt complexes.

In some embodiments, where a source of a peracid is included, an organic bleach catalyst or bleach booster may be used having one of the following formulae:
(i) 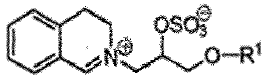
(ii) 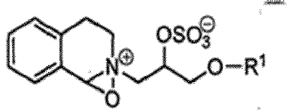
(iii) and mixtures thereof;
wherein R1 is independently a branched alkyl group containing from 9 to 24 carbons or linear alkyl group containing from 11 to 24 carbons, preferably R1 is independently a branched alkyl group containing from 9 to 18 carbons or linear alkyl group containing from 11 to 18 carbons, more preferably R1 is independently selected from the group consisting of 2-propylheptyl, 2-butyloctyl, 2-pentylnonyl, 2-hexyldecyl, dodecyl, tetradecyl, hexadecyl, octadecyl, isononyl, isodecyl, isotridecyl and isopentadecyl.

Other exemplary bleaching systems are described, e.g. in WO 2007/087258, WO 2007/087244, WO 2007/087259, EP 1 867 708 (Vitamin K) and WO 2007/087242.

### Polymers

The detergent may contain 0.005-10% by weight, such as 0.5-5%, 2-5%, 0.5-2% or 0.2-1% of a polymer. Any polymer known in the art for use in detergents may be utilized. The polymer may function as a co-builder as mentioned above, or may provide antiredeposition, fiber protection, soil release, dye transfer inhibition, grease cleaning and/or anti-foaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(ethyleneglycol) or poly(ethylene oxide) (PEG or PEO), ethoxylated poly(ethyleneimine), (carboxymethyl)inulin (CMI), carboxylate polymers and polycarboxylates such as polyacrylates, maleic/acrylic acid copolymers, acrylate/styrene copolymers, poly(aspartic) acid, and lauryl methacrylate/acrylic acid copolymers, hydrophobically modified CMC (HM-CMC), silicones, copolymers of terephthalic acid and oligomeric glycols, copolymers of poly(ethylene terephthalate) and poly(oxyethene terephthalate) (PET-POET), poly(vinylpyrrolidone) (PVP), poly(vinylimidazole) (PVI), poly(vinylpyridine-*N*-oxide) (PVPO or PVPNO) and copoly(vinylimidazole/vinylpyrrolidone) (PVPVI). Suitable examples include PVP-K15, PVP-K30, ChromaBond S-400, ChromaBond S-403E and Chromabond S-100 from Ashland Aqualon, and Sokalan^{®} HP 165, Sokalan^{®} HP 50 (Dispersing agent), Sokalan^{®} HP 53 (Dispersing agent), Sokalan^{®} HP 59 (Dispersing agent), Sokalan^{®} HP 56 (dye transfer inhibitor), Sokalan^{®} HP 66 K (dye transfer inhibitor) from BASF. Further exemplary polymers include sulfonated polycarboxylates, polyethylene oxide and polypropylene oxide (PEO-PPO) and diquaternium ethoxy sulfate. Particularly preferred polymer is ethoxylated homopolymer Sokalan^{®} HP 20 from BASF, which helps to prevent redeposition of soil in the wash liqor. Further exemplary polymers include sulfonated polycarboxylates, ethylene oxide-propylene oxide copolymers (PEO-PPO), copolymers of PEG with and vinyl acetate, and diquaternium ethoxy sulfate or quaternized sulfated ethoxylated hexamethylenediamine. Other exemplary polymers are disclosed in, e.g., WO 2006/130575. Salts of the above-mentioned polymers are also contemplated.

### Adjunct materials

Any detergent components known in the art for use in laundry/ADW/hard surface cleaning detergents may also be utilized. Other optional detergent components include anti-corrosion agents, anti-shrink agents, anti-soil redeposition agents, anti-wrinkling agents, bactericides, binders, corrosion inhibitors, disintegrants/disintegration agents, dyes, enzyme stabilizers (including boric acid, borates, CMC, and/or polyols such as propylene glycol), fabric conditioners including clays, fillers/processing aids, fluorescent whitening agents/optical brighteners, foam boosters, foam (suds) regulators, perfumes, soil-suspending agents, softeners, suds suppressors, tarnish inhibitors, and wicking agents, either alone or in combination. Any ingredient known in the art for use in laundry/ADW/hard surface cleaning detergents may be utilized. The choice of such ingredients is well within the skill of the artisan.

### Dispersants

The detergent compositions of the present invention can also contain dispersants. In particular powdered detergents may comprise dispersants. Suitable water-soluble organic materials include the homo- or co-polymeric acids or their salts, in which the polycarboxylic acid comprises at least two carboxyl radicals separated from each other by not more than two carbon atoms. Suitable dispersants are for example described in Powdered Detergents, Surfactant science series volume 71, Marcel Dekker, Inc.

### Dye Transfer Inhibiting Agents

The detergent compositions of the present invention may also include one or more dye transfer inhibiting agents. Suitable polymeric dye transfer inhibiting agents include, but are not limited to, polyvinylpyrrolidone polymers, polyamine *N*-oxide polymers, copolymers of N-vinylpyrrolidone and *N*-vinylimidazole, polyvinyloxazolidones and polyvinylimidazoles or mixtures thereof. When present in a subject composition, the dye transfer inhibiting agents may be present at levels from about 0.0001 % to about 10%, from about 0.01% to about 5% or even from about 0.1% to about 3% by weight of the composition.

### Fluorescent whitening agent

The detergent compositions of the present invention will preferably also contain additional components that may tint articles being cleaned, such as fluorescent whitening agent or optical brighteners. Where present the brightener is preferably at a level of about 0.01% to about 0.5%. Any fluorescent whitening agent suitable for use in a laundry detergent composition may be used in the composition of the present invention. The most commonly used fluorescent whitening agents are those belonging to the classes of diaminostilbene-sulfonic acid derivatives, diarylpyrazoline derivatives and bisphenyl-distyryl derivatives. Examples of the diaminostilbene-sulfonic acid derivative type of fluorescent whitening agents include the sodium salts of: 4,4'-bis-(2-diethanolamino-4-anilino-s-triazin-6-ylamino) stilbene-2,2'-disulfonate, 4,4'-bis-(2,4-dianilino-s-triazin-6-ylamino) stilbene-2.2'-disulfonate, 4,4'-bis-(2-anilino-4-(*N*-methyl-*N*-2-hydroxy-ethylamino)-s-triazin-6-ylamino) stilbene-2,2'-disulfonate, 4,4'-bis-(4-phenyl-1,2,3-triazol-2-yl)stilbene-2,2'-disulfonate and sodium 5-(2*H*-naphtho[1,2-*d*][1,2,3]triazol-2-yl)-2-[(*E*)-2-phenylvinyl]benzenesulfonate. Preferred fluorescent whitening agents are Tinopal DMS and Tinopal CBS available from Ciba-Geigy AG, Basel, Switzerland. Tinopal DMS is the disodium salt of 4,4'-bis-(2-morpholino-4-anilino-s-triazin-6-ylamino) stilbene-2,2'-disulfonate. Tinopal CBS is the disodium salt of 2,2'-bis-(phenyl-styryl)-disulfonate. Also preferred are fluorescent whitening agents is the commercially available Parawhite KX, supplied by Paramount Minerals and Chemicals, Mumbai, India. Other fluorescers suitable for use in the invention include the 1-3-diaryl pyrazolines and the 7-alkylaminocoumarins.

Suitable fluorescent brightener levels include lower levels of from about 0.01, from 0.05, from about 0.1 or even from about 0.2 wt % to upper levels of 0.5 or even 0.75 wt%.

### Soil release polymers

The detergent compositions of the present invention may also include one or more soil release polymers which aid the removal of soils from fabrics such as cotton and polyester based fabrics, in particular the removal of hydrophobic soils from polyester based fabrics. The soil release polymers may for example be nonionic or anionic terephthalte based polymers, polyvinyl caprolactam and related copolymers, vinyl graft copolymers, polyester polyamides see for example Chapter 7 in Powdered Detergents, Surfactant science series volume 71, Marcel Dekker, Inc. Other types of soil release polymers are amphiphilic alkoxylated grease cleaning polymers comprising a core structure and a plurality of alkoxylate groups attached to that core structure. The core structure may comprise a polyalkylenimine structure or a polyalkanolamine structure as described in detail in WO 2009/087523 (hereby incorporated by reference). Furthermore, random graft co-polymers are suitable soil release polymers. Suitable graft co-polymers are described in more detail in WO 2007/138054, WO 2006/108856 and WO 2006/113314 (hereby incorporated by reference). Other soil release polymers are substituted polysaccharide structures especially substituted cellulosic structures such as modified cellulose deriviatives such as those described in EP 1867808 or WO 2003/040279 (both are hereby incorporated by reference). Suitable cellulosic polymers include cellulose, cellulose ethers, cellulose esters, cellulose amides and mixtures thereof. Suitable cellulosic polymers include anionically modified cellulose, nonionically modified cellulose, cationically modified cellulose, zwitterionically modified cellulose, and mixtures thereof. Suitable cellulosic polymers include methyl cellulose, carboxy methyl cellulose, ethyl cellulose, hydroxyl ethyl cellulose, hydroxyl propyl methyl cellulose, ester carboxy methyl cellulose, and mixtures thereof.

### Anti-redeposition agents

The detergent compositions of the present invention may also include one or more anti-redeposition agents such as carboxymethylcellulose (CMC), polyvinyl alcohol (PVA), polyvinylpyrrolidone (PVP), polyoxyethylene and/or polyethyleneglycol (PEG), homopolymers of acrylic acid, copolymers of acrylic acid and maleic acid, and ethoxylated polyethyleneimines. The cellulose based polymers described under soil release polymers above may also function as anti-redeposition agents.

Other suitable adjunct materials include, but are not limited to, anti-shrink agents, anti-wrinkling agents, bactericides, binders, carriers, dyes, enzyme stabilizers, fabric softeners, fillers, foam regulators, hydrotropes, perfumes, pigments, and sod suppressors.

Further embodiments of the invention include:
Embodiment 1. A particle comprising
   (a) a protein;
   (b) a salt of sulfite, bisulfite, or metabisulfite; and
   (c) a mono- or disaccharide which is a reducing sugar or can be degraded to a reducing sugar.
Embodiment 2. The particle of the preceding embodiment, which comprises the mono- or disaccharide in an amount of more than 0.01% w/w.
Embodiment 3. The particle of any of the preceding embodiments, which comprises the mono- or disaccharide in an amount of more than 0.05% w/w.
Embodiment 4. The particle of any of the preceding embodiments, which comprises the mono- or disaccharide in an amount of more than 0.1% w/w.
Embodiment 5. The particle of any of the preceding embodiments, wherein the mono- or disaccharide is glucose, fructose and/or sucrose.
Embodiment 6. The particle of any of the preceding embodiments, wherein the mono- or disaccharide is a reducing sugar; preferably the amount of reducing sugar is measured as glucose equivalents using the PAHBAH assay.
Embodiment 7. The particle of any of the preceding embodiments, wherein the mono- or disaccharide is a monosaccharide.
Embodiment 8. The particle of any of the preceding embodiments, wherein the mono- or disaccharide is glucose, fructose, lactose, and/or maltose.
Embodiment 9. The particle of any of the preceding embodiments, wherein the mono- or disaccharide is glucose and/or fructose.
Embodiment 10. The particle of any of the preceding embodiments, which further comprises less than 0.5% w/w of titanium dioxide.
Embodiment 11. A particle comprising
   (a) a protein;
   (b) a salt of sulfite, bisulfite, or metabisulfite; and
   (c) less than 0.5% w/w of titanium dioxide.
Embodiment 12. The particle of any of the preceding embodiments, which comprises the salt of sulfite, bisulfite, or metabisulfite in an amount of more than 0.01% w/w,
Embodiment 13. The particle of any of the preceding embodiments, which comprises the salt of sulfite, bisulfite, or metabisulfite in an amount of more than 0.05% w/w.
Embodiment 14. The particle of any of the preceding embodiments, which comprises the salt of sulfite, bisulfite, or metabisulfite in an amount of more than 0.1% w/w.
Embodiment 15. The particle of any of the preceding embodiments, which comprises the salt of sulfite, bisulfite, or metabisulfite in an amount of less than 10% w/w.
Embodiment 16. The particle of any of the preceding embodiments, which comprises the salt of sulfite, bisulfite, or metabisulfite in an amount of less than 5% w/w.
Embodiment 17. The particle of any of the preceding embodiments, which comprises the salt of sulfite, bisulfite, or metabisulfite in an amount of less than 1% w/w.
Embodiment 18. The particle of any of the preceding embodiments, which comprises the protein in an amount of 0.1-25% w/w.
Embodiment 19. The particle of any of the preceding embodiments, which comprises the protein in an amount of 0.5-25% w/w.
Embodiment 20. The particle of any of the preceding embodiments, which comprises the protein in an amount of 1-25% w/w.
Embodiment 21. The particle of any of the preceding embodiments, wherein the protein is produced by fermentation.
Embodiment 22. The particle of any of the preceding embodiments, wherein the protein and the salt of sulfite, bisulfite, or metabisulfite are homogenously mixed in a continuous matrix.
Embodiment 23. The particle of the preceding embodiment, which comprises a core and a coating, and the core comprises, preferably consists of, the continuous matrix.
Embodiment 24. The particle of any of the preceding embodiments, wherein the protein is an enzyme, and the amount of protein is active enzyme protein (AEP).
Embodiment 25. The particle of any of the preceding embodiments, wherein the salt of sulfite, bisulfite, or metabisulfite is a salt of metabisulfite.
Embodiment 26. The particle of any of the preceding embodiments, wherein the salt of sulfite, bisulfite, or metabisulfite is sodium metabisulfite or potassium metabisulfite.
Embodiment 27. The particle of any of the preceding embodiments, which further comprises less than 0.1% w/w of titanium dioxide.
Embodiment 28. The particle of any of the preceding embodiments, which comprises no titanium dioxide.
Embodiment 29. The particle of any of the preceding embodiments, which exhibit reduced loss of whiteness after storage; preferably having a delta Hunter whiteness of less than 5 after storage for 8 weeks at 37°C and 70% RH.
Embodiment 30. The particle of any of the preceding embodiments, having a Hunter whiteness, which is more than 5 units higher after storage for 8 weeks at 37°C and 70% RH, as compared to the same particle comprising no salt of sulfite, bisulfite, or metabisulfite.
Embodiment 31. A method for preparing the particle of any of the preceding embodiments, comprising
   (a) preparing a homogenous mixture of a protein produced by fermentation, and a salt of sulfite, bisulfite, or metabisulfite; and
   (b) preparing a particle from the homogenous mixture.
Embodiment 32. A method for improving the whiteness after storage of a protein particle, comprising
   (a) preparing the particle of any of the preceding embodiments; and
   (b) storing the particle for more than 2 weeks.
Embodiment 33. A powder detergent comprising a surfactant and a builder, and the particle of any of the preceding embodiments.

### EXAMPLES

Chemicals were commercial products of at least reagent grade.

### Whiteness

The granulate color was measured on a HunterLab ColorFlex sensor (Model 45°/0°) using the Hunter L,a,b scale well known in the art.

Whiteness of the particles is measured as the Hunter whiteness index, WI_{H} = L-3b. The Hunter whiteness index is measured just after producing the particles (initial), and after 8 weeks storage.

### Reducing sugar assay (PAHBAH)

Reagent:
- Bismuth(III) acetate: 0.55 g/L
- Potassium sodium tartrate tetrahydrate: 5.0 g/L
- PAHBAH (4-Hydroxybenzhydrazide): 1.5 g/L
- 2% w/w NaOH: ad 100%

Diluent for samples and reference (blank):
- Deionized water

Reaction:
- 100 parts sample (diluted appropriately with diluent)
- 75 parts reagent
- Incubate at 60°C for 30 minutes
- Read optical density at 405 nm (OD405) with spectrophotometer

References:
- Glucose (read 5 points by 2-fold dilutions, e.g. 20 - 10 - 5 - 2.5 and 1.25 ppm glucose) = OD405 (glucose + reagent)
- Repeat the dilutions using diluent as reagent. This value is subtracted from the glucose above readings = OD405 (glucose + blank)
- Blank diluent using reagent (signal subtracted from glucose references and all samples) = OD405 (blank + reagent)

Samples:
- Dilute by 2-folds until at least 3 points are within the standard curve from glucose = OD405 (sample + reagent)
- Repeat the dilutions using diluent as reagent. This value is subtracted from the sample readings = OD405 (sample + blank)

Calculated data:
- Glucose signal: OD405 (glucose + reagent) - OD405 (glucose + blank) - OD405 (blank + reagent)
- Sample signal: OD405 (sample + reagent) - OD405 (sample + blank) - OD405 (blank + reagent)
- A straight line (concentration vs. signal) is fitted through the glucose signals and the sample signals (and through 0.0)
- From the slopes the amount of reducing sugars (as glucose equivalents in ppm) of the sample is calculated.

### EXAMPLE 1

### Reduced browning of enzyme particles

Enzyme particles/granules were prepared by the procedure described in Example 1 of WO 2011/134809 using high shear mixer and fluid bed coating. The dust reducing film of PEG 4000 and kaolin was applied in a mixer as described in Example 22 of US 4,106,991.

**Table 1.**

| | Uncoated | | | | | | | |
|---|---|---|---|---|---|---|---|---|
| | A1 | A2 | B1 | B2 | C1 | C2 | D1 | D2 |
| Enzyme | Lipase | Lipase | Protease | Protease | Protease Amylase Lipase Cellulase | Protease Amylase Lipase Cellulase | Protease | Protease |
| Additive | MgSO₄ | MgSO₄ | | | MgSO₄ Sodium citrate | MgSO₄ Sodium citrate | MgSO₄ Sodium citrate | MgSO₄ Sodium citrate |
| Binder | Dextrin | Dextrin | Maltitol | Maltitol | Dextrin | Dextrin | Gum arabic | Gum arabic |
| Sodium metabisulfite | | 1.0% | | 1.0% | | 1.0% | | 1.0% |
| Sodium thiosulfate | | | 0.5% | | | | 1.0% | |
| Sieving | 300-1120 microns | | | | | | | |

**Table 2. Coated particles prepared by applying a coating to the uncoated particles in Table 1.**

| | Coated (% w/w of uncoated particle) | | | | | | | |
|---|---|---|---|---|---|---|---|---|
| | A1C | A2C | B1C | B2C | C1C | C2C | D1C | D2C |
| Na₂SO₄ | 40% | 40% | 40% | 40% | 40% | 40% | 25% | 25% |
| PEG4000 | 1.5% | 1.5% | 1.5% | 1.5% | 1.5% | 1.5% | 1.5% | 1.5% |
| Kaolin | 3.6% | 3.6% | 3.6% | 3.6% | 3.6% | 3.6% | 3.6% | 3.6% |
| Sieving | 300-1200 microns | | | | | | | |
| AEP (% w/w of coated particle) | 2.2% | 2.2% | 6.8% | 6.8% | 7.2% | 7.2% | 14% | 14% |

Both coated and uncoated particles were stored in open glasses for 8 weeks at 37°C and 70% relative humidity.

**Table 3. Glucose equivalents in ppm of uncoated particles.**

| | A1 | A2 | B1 | B2 | C1 | C2 | D1 | D2 |
|---|---|---|---|---|---|---|---|---|
| Initial | 2792 | 3515 | 255 | 235 | 7613 | 6063 | 221 | 196 |
| After 8 weeks | 3510 | - | 462 | - | - | - | - | - |

The level of reducing sugars increases during storage due to break-down of non-reducing sugars.

**Table 4. Hunter whiteness of uncoated particles (A2, B2, C2 and D2 contain metabisulfite).**

| | A1 | A2 | B1 | B2 | C1 | C2 | D1 | D2 |
|---|---|---|---|---|---|---|---|---|
| Initial | 46 | 45 | 33 | 38 | 35 | 38 | 37 | 39 |
| After 8 weeks | 37 | 44 | 21 | 34 | 6 | 30 | 29 | 37 |
| Difference (delta) | 9 | 1 | 12 | 4 | 29 | 8 | 8 | 2 |

**Table 5. Hunter whiteness of coated particles (A2C, B2C, C2C and D2C contain metabisulfite).**

| | A1C | A2C | B1C | B2C | C1C | C2C | D1C | D2C |
|---|---|---|---|---|---|---|---|---|
| Initial | 41 | 42 | 39 | 41 | 36 | 39 | 36 | 38 |
| After 8 weeks | 31 | 43 | 29 | 39 | 9 | 30 | 29 | 37 |
| Difference (delta) | 10 | -1 | 10 | 2 | 25 | 9 | 7 | 1 |

It is clear from Tables 4 and 5 that the reduction (delta) in Hunter whiteness after storage is lower when the particles contain metabisulfite. Thus, addition of metabisulfite reduces browning of the particles after storage. Addition of thiosulfate does not have the same effect (see B1, D1, B1C and D1C).

## Claims

1. A particle comprising
(a) a protein;
(b) a salt of sulfite, bisulfite, or metabisulfite; and
(c) a mono- or disaccharide which is a reducing sugar or can be degraded to a reducing sugar.

2. The particle of claim 1, which comprises the mono- or disaccharide in an amount of more than 0.01% w/w; preferably more than 0.05% w/w.

3. A particle comprising
(a) a protein;
(b) a salt of sulfite, bisulfite, or metabisulfite; and
(c) less than 0.5% w/w of titanium dioxide; preferably less than 0.1% w/w of titanium dioxide.

4. The particle of any of the preceding claims, which comprises the salt of sulfite, bisulfite, or metabisulfite in an amount of more than 0.01% w/w; preferably more than 0.05% w/w.

5. The particle of any of the preceding claims, which comprises the protein in an amount of 0.1-25% w/w.

6. The particle of any of the preceding claims, wherein the protein is produced by fermentation.

7. The particle of any of the preceding claims, wherein the protein and the salt of sulfite, bisulfite, or metabisulfite are homogenously mixed in a continuous matrix.

8. The particle of claim 7, which comprises a core and a coating, and the core comprises, preferably consists of, the continuous matrix.

9. The particle of any of the preceding claims, wherein the protein is an enzyme, and the amount of protein is active enzyme protein.

10. The particle of any of the preceding claims, wherein the salt of sulfite, bisulfite, or metabisulfite is a salt of metabisulfite; preferably sodium metabisulfite or potassium metabisulfite.

11. The particle of any of the preceding claims, which further comprises less than 0.5% w/w of titanium dioxide; preferably less than 0.1% w/w of titanium dioxide.

12. The particle of any of the preceding claims, which exhibit reduced loss of whiteness after storage; preferably having a delta Hunter whiteness of less than 5 after storage for 8 weeks at 37°C and 70% RH.

13. A method for preparing a particle of any of the preceding claims, comprising
(a) preparing a homogenous mixture of a protein produced by fermentation, and a salt of sulfite, bisulfite, or metabisulfite; and
(b) preparing a particle from the homogenous mixture.

14. A method for improving the whiteness after storage of a protein particle, comprising
(a) preparing the particle of any of the preceding claims; and
(b) storing the particle for more than 2 weeks.

15. A powder detergent comprising a surfactant and a builder, and the particle of any of claims 1-12.
